# EUROPÄISCHE PATENTANMELDUNG

(11) **EP 2 418 010 A1**
(43) Veröffentlichungstag der Anmeldung: **15.02.2012**
(21) Anmeldenummer: 10008378.1
(22) Anmeldetag: 11.08.2010
(51) Int. Cl.: B01D 53/02, B01D 53/62, A61M 16/22, A62B 19/00

(54) **Vorrichtung und Verfahren zur Abreicherung von sauren Gasen aus Gasgemischen**

(71) Anmelder: Dräger Safety AG & Co. KGaA, 23560 Lübeck (DE)
(72) Erfinder: Kosegarten, Annette, 23628 Krummesse (DE)
(74) Vertreter: Schupfner, Georg

(57) **Zusammenfassung**

Gegenstand der Erfindung ist eine Vorrichtung (1) und ein Verfahren zur Abreicherung von sauren Gasen, insbesondere CO₂, aus Gasgemischen (3), insbesondere Atemgas, unter Verwendung von Hydroxid-Verbindungen (6) und Feuchtigkeit aufnehmenden Substanzen in Form von superabsorbierendem Polymer oder Vermiculit (7).

## Beschreibung

Die Erfindung betrifft eine Vorrichtung und ein Verfahren zur Abreicherung von sauren Gasen, insbesondere CO₂, aus Gasgemischen, insbesondere Atemgas unter Verwendung von Hydroxid-Verbindungen und Feuchtigkeit aufnehmenden Substanzen.

Geschlossene und halbgeschlossene Atemkreisläufe dienen dazu, dem Anwender eine von der Umgebungsluft ganz oder teilweise unabhängige Atmung zu ermöglichen. Dies ist zum Beispiel bei Arbeitskreislaufgeräten der Feuerwehren, für Gruben, aber auch Fluchtgeräten, Tauchgeräten oder U-Booten der Fall. Durch die Führung der Atemluft im Kreislauf kann Atemgas gespart werden. Dies ist insbesondere wichtig für die Anwendung in der Anästhesie. Es ist allerdings erforderlich, das vom Anwender produzierte und ausgeatmete Kohlendioxid abzureichern.

Das Abreichern des Kohlendioxids erfolgt in der Regel durch Alkali- oder Erdalkalihydroxide mit verschiedensten Beimengungen. Zum Beispiel gemäß folgender Reaktionsschemata:

CO₂ + H₂O -> H₂CO₃

H₂CO₃ + 2 NaOH -> Na₂CO₃ + 2 H₂O

Na₂CO₃ + Ca(OH)₂ -> CaCO₃ + 2 NaOH

Ca(OH)₂ + CO₂ -> CaCO₃ + H₂O + Wärme ΔHR = -113 kJ/mol

So eingesetzte Erdalkalihydroxide werden häufig als Atemkalk bezeichnet. Neben Erdalkalihydroxiden ist Wasser Reaktand, welches für eine optimale Reaktionsfähigkeit des CO₂-Absorbers benötigt wird.

Für die Anwendung in der Anästhesie sind bereits verschiedene Absorptionsmaterialien vorgeschlagen worden. Bekannte Absorptionsmaterialien sind häufig Erdalkalichloride (EP 0939671 B1, WO 98/23370), einfache Silikate oder auch Erdalkalisulfate (EP 0939671 B1). Für die Zwecke der vorliegenden Erfindung ist es aber erforderlich, ein höheres Aufnahmevermögen für Kohlendioxid, insbesondere bei Verwendung feuchter Gase, zu erzielen.

Hierbei wurde gefunden dass die Zugabe herkömmlicher Feuchthaltemittel zu Verlusten in der Aufnahmeleistung der Absorber für saure Gase und zu einer Verringerung der Abriebfestigkeit führen kann.

Bei der Lagerung von Atemkalk kann Wasser durch die Verpackung verloren gehen. Dadurch kann die CO₂-Aufnahmeleistung sinken, da das Wasser nicht mehr in ausreichender Menge als Reaktionsvermittler (vergleiche obiges Reaktionsschemata) zur Verfügung steht. Während des Transports in der jeweiligen Verpackung kann es zusätzlich zu Abrieb (Staub) des Atemkalkes kommen. Dieser Abrieb ist unerwünscht, da er sich ohne geeignete Gegenmaßnahmen im Atemkreislauf befindet und aufgenommen werden kann.

Der Erfindung liegt die Aufgabe zugrunde, Absorptionsmaterial für saure Gase zur Verfügung zu stellen, das die Nachteile des Standes der Technik nicht aufweist und insbesondere einerseits Wasser bereitstellt und andererseits Wasser bindet und lagerstabil ist, d.h. die Absorptionsfähigkeit des Absorbers über die Lagerzeit aufrecht erhalten bleibt, ohne zu Verlusten in der CO₂-Aufnahmeleistung zu führen. Eine gleichzeitige Erhöhung der Abriebfestigkeit ist weiterhin wünschenswert.

Gelöst wird diese Aufgabe durch den Gegenstand der unabhängigen Patentansprüche. Bevorzugte Ausführungsformen sind Gegenstand der Unteransprüche oder nachfolgend beschrieben.

Gegenstand der Erfindung ist die gleichzeitige Verwendung zumindest einer Hydroxid-Verbindung als Absorber für das Sauergas eines Gasgemisches und zumindest einer Wasser absorbierenden Substanz in Form eines superabsorbierenden Polymers (SAP) und/oder Vermiculit, wobei Hydroxid-Verbindung und Wasser absorbierende Substanz in Form eines superabsorbierenden Polymers und/oder Vermiculit miteinander im unmittelbaren räumlichen Kontakt stehen. Sauergas ist insbesondere Kohlendioxid (CO₂). Das Gasgemisch ist insbesondere Umgebungsluft, vorzugsweise Atemgas, z.B. solches wie es durch einen Ausatemvorgang bereitgestellt wird.

Geeignete Hydroxid-Verbindungen sind Erdalkalihydroxide, insbesonder Calciumhydroxid, oder Erdalkalihydroxide zusammmen mit Alkalihydroxiden wie Calciumhydroxid zusammen mit Natrium- und/oder Lithiumhydroxid. Die Alkalihydroxide werden bevorzugt zu geringeren Anteilen (Massenanteile) als die Erdalkalihydroxide eingesetzt.

Die Wasser absorbierenden Substanzen, vorliegend auch Feuchtebindemittel genannt, sind superabsorbierende Polymere (SAP) oder ein Vermiculit (Schichtsilikat). Diese wirken einerseits als Feuchthaltemittel und andererseits weisen diese eine hohe Feuchtebindefähigkeit auf.

Superabsorbierende Polymere im Sinne der vorliegenden Erfindung sind Polymere, die in der Lage sind, ein Vielfaches ihres Eigengewichts an Wasser aufzusaugen. Geeignete Superabsorber sind z.B. Copolymere aus Acrylsäure und Acrylatsalzen (z.B. Natriumacrylat), hergestellt unter Verwendung von Vernetzungsmitteln (z.B. Core-Cross-Linker). Für die vernetzten Polyacrylsäuren (crosslinked) können unterschiedliche Vernetzungsmittel zum Einsatz kommen. Durch die Vernetzung der Polymerketten wird das Polymer wasserunlöslich. Wasser dringt in den Polymerpartikel ein, so dass dieser aufquillt und das Wasser bindet. Weitere superabsorbierende Polymere sind vernetzte Polysaccharid-Derivate,

Unter superabsorbierenden Polymeren werden im Rahmen dieser Erfindung vernetzte, organische Polymere verstanden, die in Wasser zwar quellbar, aber nicht löslich sind. Sie quellen mit Wasser auf ein Vielfaches ihres Eigengewichtes an. Geeignete superabsorbierende Polymere umfassen chemisch gesehen teilneutralisierte und vernetzte Polyacrylsäuren, (Teil)-Hydrolysate von Stärke-Acrylnitril-Pfropfcopolymeren, (teil-)neutralisierte Stärke-Acrylsäure Pfropfcopolymere, (teil)verseifte Vinylacetat-Acrylsäureester-Copolymere, (teil-)hydrolysierte Acrylnitriloder Acrylamidcopolymere, vernetzte Produkte solcher Hydrolysate und Polymere aus vernetzten kationischen Monomeren. Im einzelnen können in den vernetzten superabsorbierenden Polymeren folgende Monomere allein oder in Kombination enthalten sein: Acrylsäure, Methacrylsäure, Vinylsulfonsäure, Styrolsulfonsäure, 2-(Meth)acrylamid-2-methylpropansulfonsäure, 2-(Meth)acryloylethansulfonsäure, 2-(Meth)acryloylpropansulfonsäure sowie die Salze der zuvor genannten Säuren. Ferner (Meth)-acrylamid, N-ethyl-(meth)acrylate, N,N-dimethylaminopropyl-(meth)acrylate, N,N-dimethylaminopropyl(meth)acrylamide sowie deren quaternäre Salze und Vinylpyrrolidon. Als Vernetzer eignen sich beispielsweise Allylmethacrylat, Diethylenglykoldiacrylat, ethoxyliertes Trimethylolpropantriacrylat, Ethylenglykoldiglycidylether, Methylenbisacrylamid, Tetraallyloxyethan, Triallylamin und Trimethylolpropantriacrylat. Weitere Angaben über Superabsorber sind dem Buch " Modern Superabsorbent Polymer Technology", herausgegeben von Fredric L. Buchholz und Andrew T. Graham, Verlag Wiley - VCH (1998) zu entnehmen.

Bevorzugt sind superabsorbierende Polymere auf Basis von Polyacrylat, Polymethacrylat sowie entsprechenden Copolymerisaten.

Vermiculit ist ein Schichtsilikat und kann durch die allgemeine Zusammensetzung:

(Mg_{0,5},Ca₀,₅,Na,K)₀,₇(Mg,Fe,Al)₃[(OH)_{2]}(Al,Si)₂Si₂O₁₀] . 4H₂O

beschrieben werden.

Superabsorber können, je nach Formulierung, bis zu dem 1000-fachen ihres Eigengewichtes an Wasser aufnehmen. Vermiculit immerhin noch bis zu dem 20-fachen, während herkömmliche Feuchthaltemittel, wie zum Beispiel Calciumchlorid, nur etwa das Doppelte ihres Eigengewichts an Feuchtigkeit aufnehmen können.

Der Absorber aufweisend die Absorbermasse enthält bezogen auf den Absorber vorzugsweise größer 10 Gew.% , besonders bevorzugt größer 15 Gew%. und ggf. auch größer 20 Gew.% Wasser. Der Beladungsgrad mit Wasser in Gew.% kann z.B. mittels des durch Trocknung bei erhöhten Temperaturen erhaltenen Differenzwertes bestimmt werden.

Als Absorbermasse wird im Sinne der vorliegenden Erfindung abschließend die Summe der/den Hydroxid-Verbindungen, der/den superabsorbierendem Polymer(en) und dem Vermiculit verstanden. Der Absorber kann im Unterscheid außer der Absorbermasse weitere Bestandteile enthalten.

Nach einer Ausgestaltung machen obige Wasser absorbierenden Substanzen (superabsorbierendes Polymer(en) und/oder Vermiculit aber ohne Wasser) 0,5 bis 15 Gew.%, insbesondere 0,5 bis 5 Gew.%, der Absorbermasse (100 Gew.%) aus. Den Rest bis auf 100 Gew.% bilden die Hydroxid-Verbindung.

Die Erfindung ist weiterhin gekennzeichnet durch den Aufbau des Absorbers. Das Feuchthaltemittel steht im Absorber, z.B. in Form eines Absorberbettes, mit der Hydroxid-Verbindung in Kontakt, vorzugsweise im allseitigen Kontakt:
- alle Bestandteile können gemeinsam in Form eines Pulvers oder Granulates vorliegen (Schüttung).
- Feuchthaltemittel und Hydroxid-Verbindung sind jeweils in Pulver- oder Granulatform in Schichten neben- /aufeinander angeordnet, die z.B. durch ein Gitterstruktur wie ein Vlies voneinander getrennt sind.
- Hydroxid-Verbindung und/oder Wasser absorbierende Substanz sind gemeinsam, ggf. auch in Schichten, auch auf einem Trägermaterial, wie z.B. einem Vlies aufgebracht (geträgert).
- Das Feuchthaltemittel ist im Absorptionsbett um das Hydroxid herum angeordnet, etwa indem die Hydroxid-Verbindung als Granulat vorliegt und die Wasser absorbierende Substanz, insbesondere der polymere Superabsorber, auf das Granulat aufgezogen ist, oder umgekehrt.

Die Hydroxid-Verbindung kann, ggf. mit anderen Substanzen zusammen, als Granulat vorliegen, typischerweise im Korngrößenbereich zwischen 1 bis 5 mm Grundlage der Korngrößenanalyse kann z.B ein Verfahren nach der USPharmacopoeia 27 NF22 sein. Die Granulatform kann dabei unregelmäßig sein, aber auch halbkugel- oder kugelförmig oder als Röllchen oder Tablette.

Das Feuchtebindemittel kann auch um das CO₂-Absorptionsmittel herum als Granulat oder auf einem Träger angeordnet sein, wobei das Feuchtebindemittel für die Hydroxid-Verbindung Träger ist oder umgekehrt. Es ist auch möglich Hydroxid-Verbindung und Feuchtebindemittel gemeinsam zu Pellets zu verarbeiten, um mit diesen eine Schüttung herzustellen, oder beide auf feste Träger aufzuziehen. Solche Träger können eine Gitter-, Waben- oder Netzstruktur aufweisen.

Die Erfindung findet vorrangig Verwendung in Arbeitskreislaufgeräten und der Anästhesie, kann aber auch in jeder anderen Anwendung verwendet werden, bei der die heutigen Absorptionsmaterialien auf Basis von Hydroxiden Verwendung für saure Gase, wie Kohlendioxid, finden. Es werden hierbei üblicherweise Atemsysteme verwendet, bei denen das Atemgas im Kreislauf zirkuliert und nach Entfernung des ausgeatmeten Kohlendioxids dem Atmenden wieder zugeführt wird. Verbrauchtes Atemgas wird substituiert, wobei in Anästhesiesystemen volatile Anästhetika zugegeben werden.

Der Vorteil der vorliegenden Erfindung beruht darin, dass durch das Feuchtebindemittel das Wasser im Absorptionsmaterial selbst in großer Menge gebunden werden kann und somit für eine Reaktionsvermittlung mit dem sauren Gas und damit einer Verlängerung der Lagerzeit zur Verfügung steht. Im Falle der Anästhesieanwendung ist zu erwarten, dass auch keine oder eine reduzierte Narkosemittelzersetzung auftritt, da diese durch Austrocknung begünstigt wird und dies durch Zugabe des Feuchtebindemittels unterbunden wird. Außerdem wird weniger Reaktionswasser aus dem Absorptionsbett in den Atemkreislauf entlassen, so dass der Gerätebetrieb weniger beeinflusst wird und sich eine physiologisch angenehme Einatemfeuchte einstellen kann. Als positiver Nebeneffekt tritt zusätzlich auf, dass die Absorbermasse sowieso nach jedem Gebrauch verbraucht ist und gewechselt werden muss, und somit kein zusätzlicher Aufwand für den Anwender durch Leeren von Kondensatfallen oder Ähnlichem erforderlich ist. Durch die Eigenschaft der Superabsorber, Wasserstoffbrücken zu bilden, die dann das Wasser festhalten, erhöht sich auch die mechanische Beständigkeit, da gerade in Verbindung mit Wasser die Vernetzung innerhalb des Absorbers erhöht wird.

Die Erfindung bzw. deren Eigenschaften sind durch die Figuren näher erläutert. Es zeigen:
Fig. 1: Die Abrasionsneigung verschiedener Atemkalke,
Fig. 2: das CO₂-Absorbtionsvermögen verschiedener Atemkalke,
Fig. 3: eine mögliche Anordnung in einer Filterkartusche und
Fig. 4: eine andere mögliche Anordnung in einer Filterkartusche.

Obige Vorteile sind nicht jedem Wasserbindemittel gegeben, wie die folgenden Versuchsbeispiele zeigen:

### Rezepturbeispiel 1:

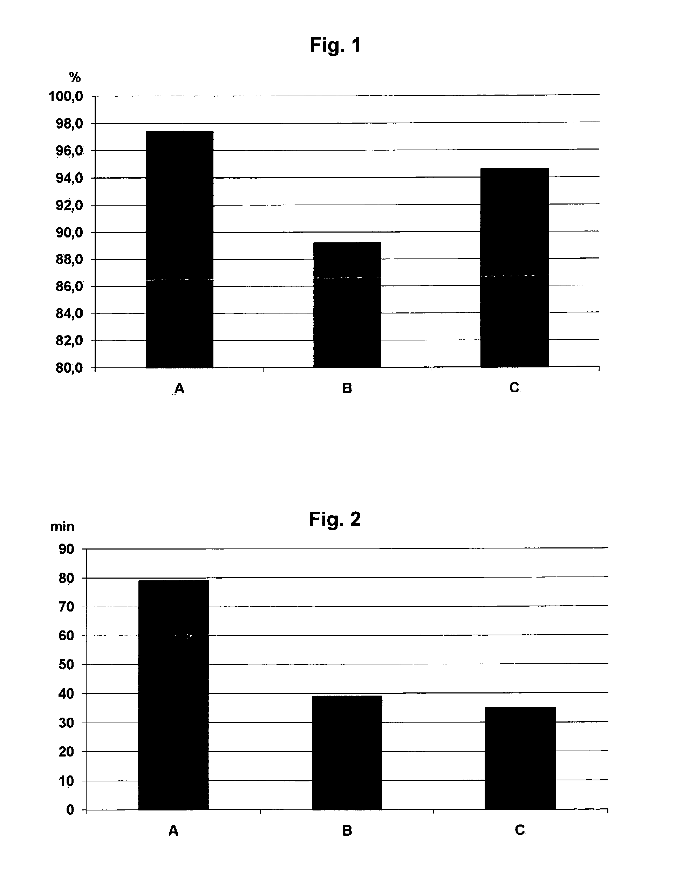

Calciumhydroxid wurde mit 3 Gew.% Natriumhydroxid und 1 Gew.% Superabsorber auf Acrylatbasis und einem Überschuss an Wasser gemischt. Anschließend wird die Paste granuliert und auf einen Wassergehalt von ca. 16 % getrocknet. Das erhaltene Produkt ist in Fig. 1 und 2 als Produkt A wiedergegeben.

Die Abriebfestigkeit wurde in einem Friabilator, wie er u.a. für Tablettenprüfungen verwendet wird, über 7 h ermittelt und ist in Fig. 1 gezeigt. Ein herkömmlicher CO₂-Absorber B enthält Calciumchlorid als Feuchtebindemittel, ein anderes herkömmliches Produkt C Silikate.

Im Fall von Produkt B und C ist die Abriebfestigkeit reduziert. Beide Atemkalke mit Bindemittel zeigen eine Verminderung der CO₂-Leistung im STANAG 1411 CO₂-Test (vergleiche Fig. 2). Bei Verwendung von Superabsorber wird die Abriebfestigkeit erhöht bei guten CO₂-Leistungen.

Der Abriebtest wurde so durchgeführt, dass 10 g staubfreier CO₂-Absorber 7 Stunden in einem Friabilator rotiert. Danach wurde über einem 0,42 mm Sieb abgesiebt. Der prozentual errechnete Abrieb (< 0,42 mm) ergab von 100 subtrahiert die Abriebfestigkeit. Der CO₂-Aufnahmetest ist in dem Nato Standard 1411 beschrieben.

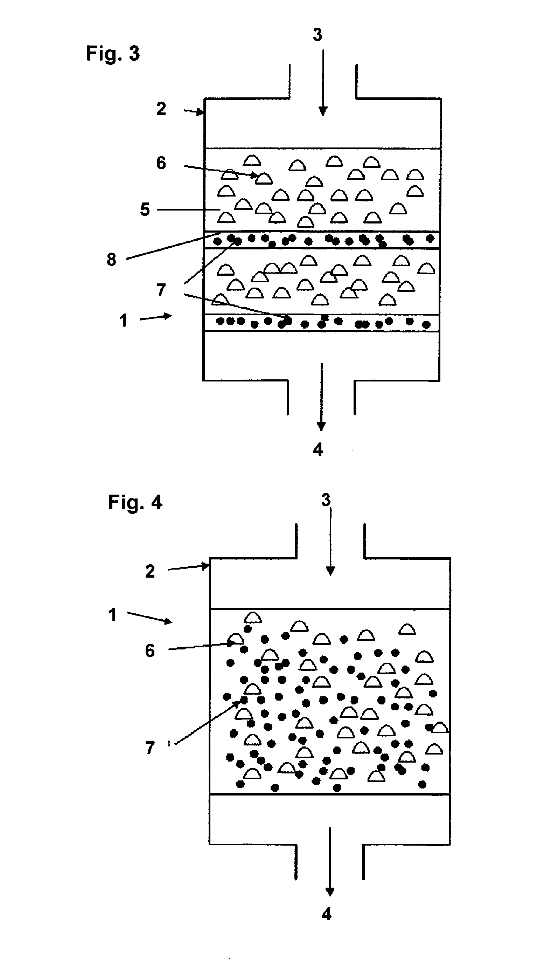

Fig. 4 zeigt schematisch den Aufbau eines CO₂-Absorbers in Form eines Filterelements, wie es in einem Atemsystem eingesetzt werden kann. Das CO₂-Filterelement 1 weist ein Gehäuse 2 mit einem Gaseinlass 3 und einem Gasauslass 4 mit einem als Absorberbett 5 ausgestalteten Absorber auf, versehen mit CO₂-Absorber 6 und Feuchtebindemittel 7 in Form einer Schüttung. In Fig. 3 ist ein Aufbau des Absorbers in Schichten gezeigt, die durch ein Vlies 8 getrennt sind

## Patentansprüche

1. Vorrichtung zur Abreicherung von sauren Gasen aus einem Gasgemisch, wobei die Vorrichtung einen Absorber umfasst, der enthält:
- Kohlendioxid aufnehmende Substanzen, aufweisend oder bestehend zumindest einer Hydroxid-Verbindung und
- Wasser absorbierende Substanzen aufweisend oder bestehend aus su-perabsorbierendem Polymer (SAP) und/oder Vermiculit, wobei
Hydroxid-Verbindung(en) einerseits und superabsorbierendes Polymer (SAP) und/oder Vermiculit andererseits die Absorbermasse bilden und unmittelbar in Kontakt miteinander stehen.

2. Vorrichtung nach Anspruch 1, **dadurch gekennzeichnet, dass** der Absorber unabhängig voneinander
- eine Schichtstruktur hat , umfassend zumindest drei unterschiedliche Schichten der Absorbermasse, wobei zumindest eine Schicht von zwei weiteren anderen Schichten zumindest teilweise umgeben ist,
- eine durchmischte Aufschüttung von Partikeln umfassend die Absorbermasse ist,
- das superabsorbierende Polymer und/oder das Vermiculit und die Hydroxid-Verbindung auf einem festen oder flexiblen Träger aufgebracht sind,
- das superabsorbierende Polymer und/oder das Vermiculit auf die Hydroxid-Verbindung als Träger aufgebracht ist oder umgekehrt und/oder
- die Hydroxid-Verbindung und das superabsorbierende Polymer und/oder das Vermiculit jeweils gemeinsam in Partikel eingearbeitet sind.

3. Vorrichtung nach zumindest einem der vorhergehenden Ansprüche, **dadurch gekennzeichnet, dass** die Hydroxid-Verbindung ein Erdalkalihydroxid ist oder umfasst.

4. Vorrichtung nach zumindest einem der vorhergehenden Ansprüche, **dadurch gekennzeichnet, dass** die Hydroxid-Verbindungen ein Erdalkalihydroxid und ein Alkalihydroxid aufweisen, vorzugsweise das Alkalihydroxid zu 0,5 bis 8 Gew.%, bezogen auf die Masse der eingesetzten Hydroxid-Verbindungen.

5. Vorrichtung nach zumindest einem der vorhergehenden Ansprüche, **dadurch gekennzeichnet, dass** die Hydroxid-Verbindung ein Natrium- und/oder Lithiumhydroxid ist oder umfasst.

6. Vorrichtung nach zumindest einem der vorhergehenden Ansprüche, **dadurch gekennzeichnet, dass** die Wasser absorbierenden Substanzen 0,5 bis 15 Gew.% der Absorbermasse ausmachen, exkl. ggf. aufgenommenem Wasser.

7. Vorrichtung nach zumindest einem der vorhergehenden Ansprüche, **dadurch gekennzeichnet, dass** der Absorber aufweisend die Absorbermasse bezogen auf den Absorber größer 10 Gew.%, besonders bevorzugt größer 15 Gew%. und ggf. auch größer 20 Gew.% Wasser enthält.

8. Atemkreislaufgerät enthaltend die Vorrichtung nach einem der vorherigen Ansprüche, wobei der Absorber insbesondere Teil einer austauschbaren oder befüllbaren Kartusche ist.

9. Verfahren zum Entfernen von sauren Gasen, insbesondere Kohlendioxid, aus einem Gasgemisch, insbesondere einem Gasstrom, unter Verwendung der Vorrichtung nach zumindest einem der Ansprüche 1 bis 7 oder des Atemkreislaufgerätes nach Anspruch 8.
